# EUROPÄISCHE PATENTSCHRIFT

(11) **EP 1 339 443 B1**
(45) Veröffentlichungstag und Bekanntmachung des Hinweises auf die Patenterteilung: **20.01.2016**
(21) Anmeldenummer: 01999398.9
(22) Anmeldetag: 16.11.2001
(51) Int. Cl.: A61M 1/10, A61M 1/12

(54) **Verfahren zum Kalibrieren eines Drucksensors oder eines Flusssensors an einer Rotationspumpe**
Method for calibrating a pressure sensor or a flow sensor on a rotation pump
Procédé pour étalonner un capteur de pression ou un capteur de débit sur une pompe rotative

(30) Priorität: 05.12.2000 DE 10060275
(43) Veröffentlichungstag der Anmeldung: 03.09.2003
(73) Patentinhaber: Abiomed Europe GmbH, 52074 Aachen (DE)
(72) Erfinder: SIESS, Thorsten, 52146 Würselen (DE); NIX, Christoph, 52222 Stolberg (DE); BÖNSCH, Stefan, 52074 Aachen (DE)
(74) Vertreter: von Kreisler Selting Werner - Partnerschaft von Patentanwälten und Rechtsanwälten mbB
(86) Internationale Anmeldenummer: PCT/EP2001/013266
(87) Internationale Veröffentlichungsnummer: WO 2002/045775

(56) Entgegenhaltungen:
- EP-A1- 0 973 082
- WO-A1-91/12035
- WO-A1-97/49439
- WO-A1-98/43688
- US-A- 6 024 704

## Beschreibung

Die Erfindung betrifft ein Verfahren zum Kalibrieren eines Drucksensors oder eines Flusssensors an einer das pulsierende Herz unterstützenden Rotationspumpe.

Es ist bekannt, zur Herzunterstützung eine intrakardiale Rotationspumpe einzusetzen. WO 98/43688 beschreibt eine intrakardiale Blutpumpe, bei der die Steuerung des Pumpenbetriebes in Abhängigkeit von dem Messsignal einer Druckmesseinrichtung erfolgt. Anhand des gemessenen Druckes kann die Förderrate der Pumpe bestimmt werden. Das aus einem Rohr bestehende Pumpengehäuse enthält in einem Fall einen Differenzdrucksensor, der den Differenzdruck zwischen Auslassdruck und Einlassdruck der Pumpe bestimmt. Der Pumpeneinlass ist mit einer Kanüle versehen, die im Falle einer Linksherzpumpe durch die Aortenklappe hindurchragt. Durch diese Kanüle entstehen zusätzliche Verluste, die weitgehend proportional zur Pumpendrehzahl sind und deren Einflüsse rechnerisch berücksichtigt werden können. Eine wesentliche Fehlerquelle besteht darin, dass am Drucksensor eine Drift auftreten kann. Eine solche Drift erfordert ein Nachkalibrieren des Drucksensors bzw. die Ermittlung eines Korrekturwertes.

WO 91/12035 A1 beschreibt ein Verfahren und eine Vorrichtung zur Durchflussregulierung einer Herzprothese mit periodischem Durchfluss.

EP 0 973 082 A1 beschreibt ein Verfahren zur Regelung des Drucks eines Fluides, das durch einen von einem elektrischen Motor angetriebenen Druckerzeuger in einem mindestens einen Verbraucher aufweisenden Rohrsystem bewegt wird.

US 6,024,704 A beschreibt einen implantierbaren barometrischen Drucksensor, der mit einem implantierbaren medizinischen Gerät verbunden ist und einen barometrischen Referenzdruck ermittelt zur Benutzung in Verbindung mit einem Absolutdruckwert, der von einem implantierbaren Absolutdrucksensor gemessen wird, welcher mit dem implantierbaren medizinischen Gerät verbunden ist.

Der Erfindung liegt die Aufgabe zugrunde, ein Verfahren zum Kalibrieren eines Drucksensors an einer das pulsierende Herz unterstützenden Rotationspumpe anzugeben, das in situ an der im Herzen befindlichen Pumpe durchgeführt werden kann, so dass es nicht erforderlich ist, die Pumpe zu entfernen.

Das erfindungsgemäße Verfahren eignet sich insbesondere für eine längerfristig implantierte Pumpe (Wochen bis Monate). Das Kalibrationsverfahren ist auch für implantierbare Pumpen anwendbar, wie sie in WO97/49439 beschrieben sind. Hierbei handelt es sich um Herzunterstützungspumpen die um das Herz herum platziert sind und mittels Ein- und Auslasskanäle an das Herz und die abführenden Gefäße angeschlossen sind. Derartige Pumpen werden auch als parakardiale Pumpen bezeichnet.

Die Lösung dieser Aufgabe erfolgt erfindungsgemäß mit den im Anspruch 1 angegebenen Merkmalen.

Die Erfindung basiert auf dem Gedanken, dass bei einer Blutpumpe, die entweder durch eine Herzklappe hindurchgeht, oder deren Einlasskanüle von der Auslasskanüle über eine natürliche Herzklappe getrennt sind, prinzipiell die Druckdifferenz immer dann zu Null wird, wenn die Herzklappe geöffnet ist. Wenn die Pumpe jedoch mit einer Kanüle versehen ist, wird bei laufender Pumpe keine Druckdifferenz von Null erreicht, weil im Zuge der Kanüle ein Druckabfall auftritt, der auf Einlaufverluste, Rohrreibungsverluste und eine dynamische Druckabsenkung Wenn die Pumpe jedoch mit einer Kanüle versehen ist, wird bei laufender Pumpe keine Druckdifferenz von Null erreicht, weil im Zuge der Kanüle ein Druckabfall auftritt, der auf Einlaufverluste, Rohrreibungsverluste und eine dynamische Druckabsenkung zurückgeht. Gleichwohl zeigt die zeitliche Kurve des Druckverlaufs bei geöffneter Herzklappe ein Minimum. Erfindungsgemäß wird eine Tabelle oder Kurve der Soll-Druckminima in Abhängigkeit von der Drehzahl der Pumpe für einen ideal driftfreien Drucksensor erstellt. Während des Pumpenbetriebes wird ein Ist-Druckminimum des Messdrucks des Drucksensors ermittelt und dieses wird mit dem Soll-Druckminimum, das der betreffenden Drehzahl zugeordnet ist, verglichen. Aus der Differenz der Druckminima wird ein Korrekturwert für die nachfolgend gemessenen Druckwerte bestimmt. Auf diese Weise kann bei laufender Rotationspumpe eine Drift des Drucksensors korrigiert werden.

Das erfindungsgemäße Verfahren wird vorzugsweise bei einem Differenzdrucksensor durchgeführt, jedoch ist es auch für die Driftkorrektur von Absolut-Drucksensoren geeignet.
- Fig. 3: ein Diagramm des zeitlichen Verlaufs verschiedener Drücke, in Relation zum linken Herzen,
- Fig. 4: den zeitlichen Verlauf des Differenzdrucks bei unterschiedlichen Drehzahl- oder Leistungsstufen der Linksherzpumpe,
- Fig. 5: ein Diagramm des am Differenzdruck gemessenen Mindestwertes der Druckdifferenz in Abhängigkeit von der Drehzahl- oder Leistungsstufe,
- Fig. 6: eine schematische Darstellung des Herzens mit einer Rechtsherzpumpe, und
- Fig. 7: ein Diagramm der Abhängigkeit des Flusses FL von dem Differenzdruck ΔP_{Herz}, wobei die Flussmaxima auf der Ordinate bei ΔP_{minHerz} = 0 liegen.

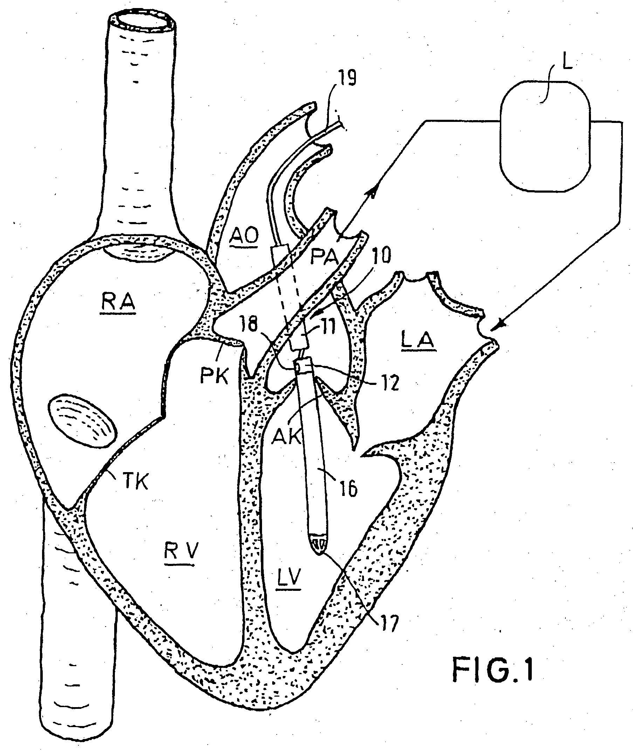

In Fig. 1 ist eine Linksherzpumpe 10 dargestellt, die von dem linken Ventrikel LV in die Aorta AO fördert und dadurch die pulsierende natürliche Pumpfunktion des Herzens unterstützt. Die Pumpe 10 ragt durch die Aortenklappe AK hindurch, ohne deren Funktion zu beeinträchtigen.

In Fig. 1 sind ferner das rechte Atrium RA, die Tricuspidalklappe TK und der rechte Ventrikel RV des Herzens dargestellt, so wie die Pulmonalklappe PK, die Pulmonalarterie PA, die zur Lunge L führt und das linke Atrium LA.

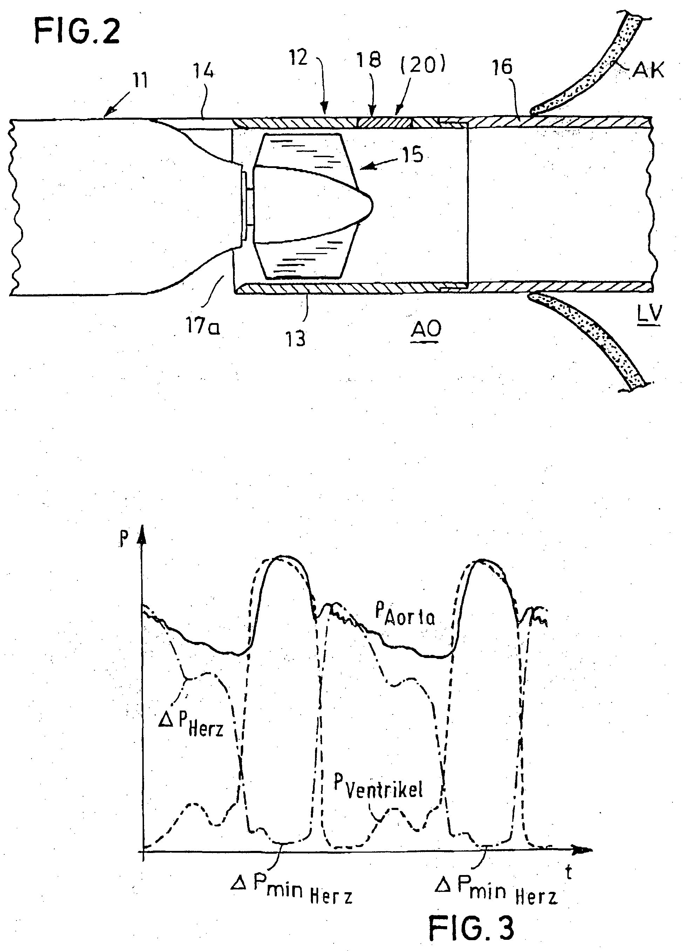

Die Pumpe 10 ist eine Rotationspumpe, wie sie generell in WO 98/43688 beschrieben ist. Sie hat einen Antriebsteil 11, der einen Elektromotor enthält und einen Pumpenteil 12. Gemäß Fig. 2 weist der Pumpenteil 12 ein rohrförmiges Pumpengehäuse 13 auf, das mit dem Antriebsteil 11 über Stege 14 verbunden ist. In dem Pumpengehäuse 13 rotiert ein Flügelrad 15, das mit der Motorwelle verbunden ist. An das Pumpengehäuse 13 schließt sich eine schlauchförmige flexible Kanüle 16 an, die an ihrem vorderen Ende mit Einlassöffnungen 17 versehen ist (Fig. 1). Die Auslassöffnung 17a der Pumpe befindet sich im Übergangsbereich zwischen dem Antriebsteil 11 und dem Pumpengehäuse 13.

Am Pumpengehäuse 13 ist ein Drucksensor 18 vorgesehen, der hier als Differenzdrucksensor ausgebildet ist und den Differenzdruck zwischen Innendruck und Außendruck des Gehäuses 13 misst. Der Drucksensor 18 ist über (nicht dargestellte) Leitungen, die durch einen Katheter 19 (Fig. 1) verlaufen, mit einem extrakorporalen Messgerät verbunden. Der Katheter 19 schließt sich an das proximale Ende des Antriebsteils 11 an. Durch ihn verlaufen auch die Versorgungsleitungen für den Elektromotor. Ein externes Steuergerät steuert die Betriebsdrehzahl des Elektromotors und bestimmt somit eine von mehreren Drehzahlstufen, mit der die Pumpe betrieben wird.

Der im Antriebsteil 11 befindliche Elektromotor wird drehzahlgeregelt betrieben. Die Druckdifferenz gibt in Verbindung mit der Drehzahl Aufschluss über den Volumenstrom der Pumpe.

Der Drucksensor 18 besteht aus einer Membran, die durch die an den beiden Membranflächen anliegenden Drücke verformt wird. Der außen anliegende Druck entspricht dem Druck des umgebenden Gefäßes (Aorta). Der innerhalb des Pumpengehäuses herrschende Druck entspricht dem Druck an der Kanülenspitze, vermindert um den Druckverlust der Kanüle und die dynamische Druckabsenkung infolge der Strömungsgeschwindigkeit. Bei Platzierung der Pumpe gemäß Fig. 1 entspricht der vom Drucksensor 18 gemessene Differenzdruck ΔP = P_{Aorta} - P_{Ventrikel} + P_{Kanüle}.

Die beiden Drücke P_{Aorta} und P_{Ventrikel} sind in Fig. 3 in ihrem zeitlichen Verlauf dargestellt. Ebenso ist der Differenzdruck ΔP_{Herz} dargestellt. Immer dann, wenn die Aortenklappe AK geöffnet ist, ist die Druckdifferenz ΔP_{Herz} nahe Null. Dieser Zustand ist mit ΔP_{minHerz} bezeichnet.

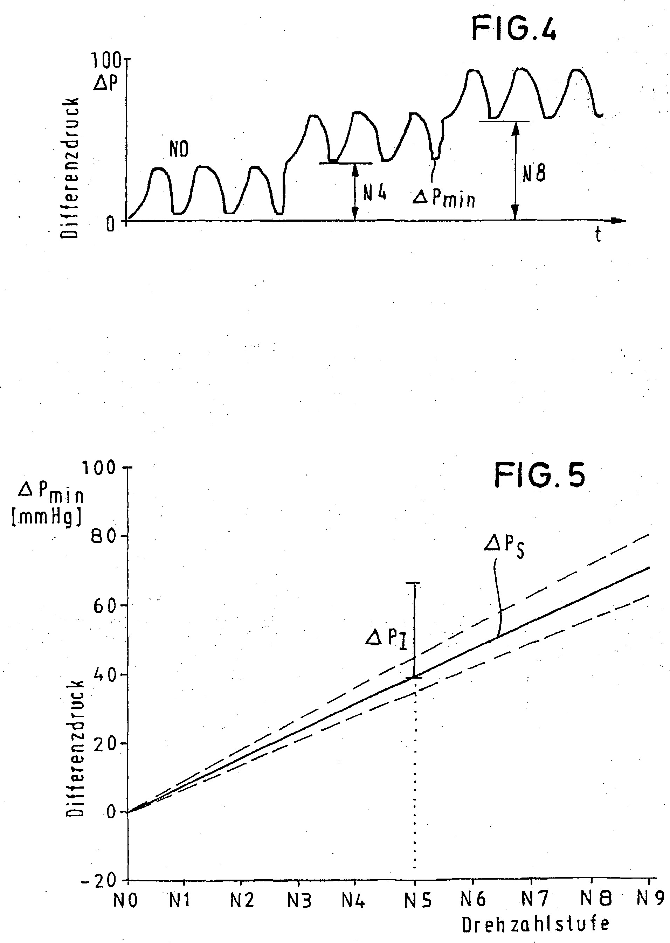

Fig. 4 zeigt den zeitlichen Druckverlauf des Differenzdrucks ΔP bei unterschiedlichen Drehzahlstufen NO, N4 und N8 der Rotationspumpe. In der Drehzahlstufe NO ist die Rotationspumpe abgeschaltet. Die durch die natürliche Herzfunktion gebildete Druckamplitude zwischen Systole und Diastole bleibt auch bei Pumpenbetrieb weitgehend erhalten. Abhängig von der eingestellten Drehzahlstufe der Pumpe verändert sich jedoch das allgemeine Druckniveau. Das Differenzdruckminimum ΔPₘᵢₙ wird um genau den Wert erhöht, der sich durch die Pumpenunterstützung einstellt. Liegt die Spitze der Kanüle 16 im linken Ventrikel LV und das Pumpengehäuse 13 in der Aorta AO stellt sich die Druckdifferenz ΔP als sinuidales Signal dar.

Fig. 5 zeigt die Höhe der Differenzdruckminima ΔPₘᵢₙ in Abhängigkeit von der Drehzahlstufe N0 bis N9. Man erkennt, dass die Höhe des Differenzdruckminimums proportional mit der Drehzahl ansteigt. Die Kurve ΔP_{S} gibt die Soll-Druckminima an. Um diese Kurve herum gibt es einen Toleranzbereich.' Liegt ein in einem bestimmten Drehzahlbereich, z.B. bei Drehzahl N5, gemessenes Ist-Druckminimum ΔP_{I} außerhalb des Toleranzbereichs, dann wird die Differenz von Ist-Druckminimum ΔP_{I} und Soll-Druckminimum ΔP_{S} derselben Drehzahl (hier: N5) gebildet. Diese Differenz allgemeine Druckniveau. Das Differenzdruckminimum ΔPₘᵢₙ wird um genau den Wert erhöht, der sich durch die Pumpenunterstützung einstellt. Liegt die Spitze der Kanüle 16 im linken Ventrikel LV und das Pumpengehäuse 13 in der Aorta AO stellt sich die Druckdifferenz ΔP als sinuidales Signal dar.

Fig. 5 zeigt die Höhe der Differenzdruckminima ΔPₘᵢₙ in Abhängigkeit von der Drehzahlstufe N0 bis N9. Man erkennt, dass die Höhe des Differenzdruckminimums proportional mit der Drehzahl ansteigt. Die Kurve ΔP_{S} gibt die Soll-Druckminima an. Um diese Kurve herum gibt es' einen Toleranzbereich. Liegt ein in einem bestimmten Drehzahlbereich, z.B. bei Drehzahl N5, gemessenes Ist-Druckminimum ΔP_{I} außerhalb des Toleranzbereichs, dann wird die Differenz von Ist-Druckminimum ΔP_{I} und Soll-Druckminimum ΔP_{S} derselben Drehzahl (hier: N5) gebildet. Diese Differenz bildet die aufgetretene Drift des Drucksensors. Sie wird zur Korrektur der anschließend gemessenen Druckwerte benutzt.

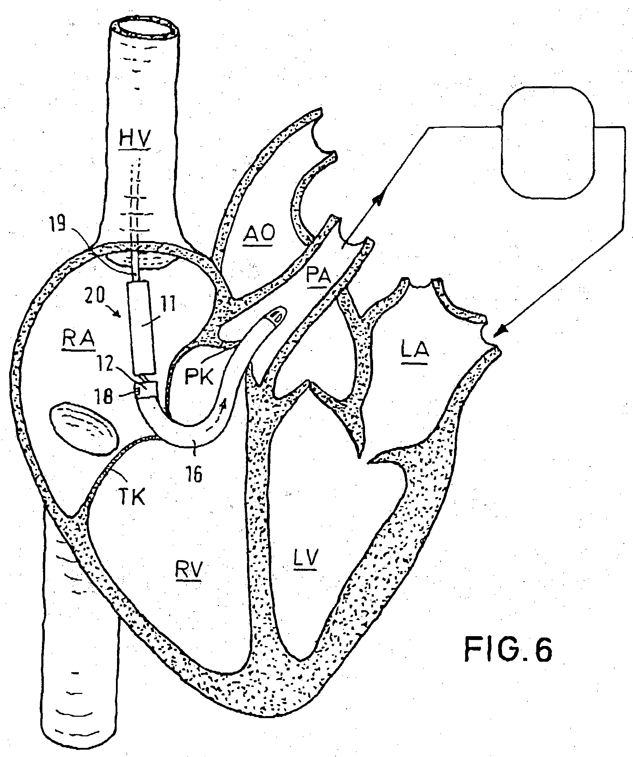

Fig. 6 zeigt eine intrakardiale Rechtsherzpumpe, die generell in gleicher Weise ausgebildet ist wie die Pumpe 10, jedoch in umgekehrter Richtung fördert. Die Pumpe 20 ist mit dem Katheter 19 durch die obere Hohlvene HV hindurch verlegt und befindet sich im rechten Atrium RA, wobei die Kanüle 16 durch die Tricuspidalklappe TK und die Pulmonalklappe PK hindurch in die Pulmonalarterie PA fördert.

Das Kalibrenen der Pumpe nach Fig. 6 erfolgt nicht nach dem erfindungsgemäßen Verfahren.

Zum Kalibrieren des Drucksensors 18 der Rechtsherzpumpe 20 wird die Pumpe 20 stillgesetzt und der Differenzdruck gemessen. Der Differenzdruck muss im Mittel 5 mmHg betragen. Weicht er von diesem Wert ab, wird anhand der Abweichung ein

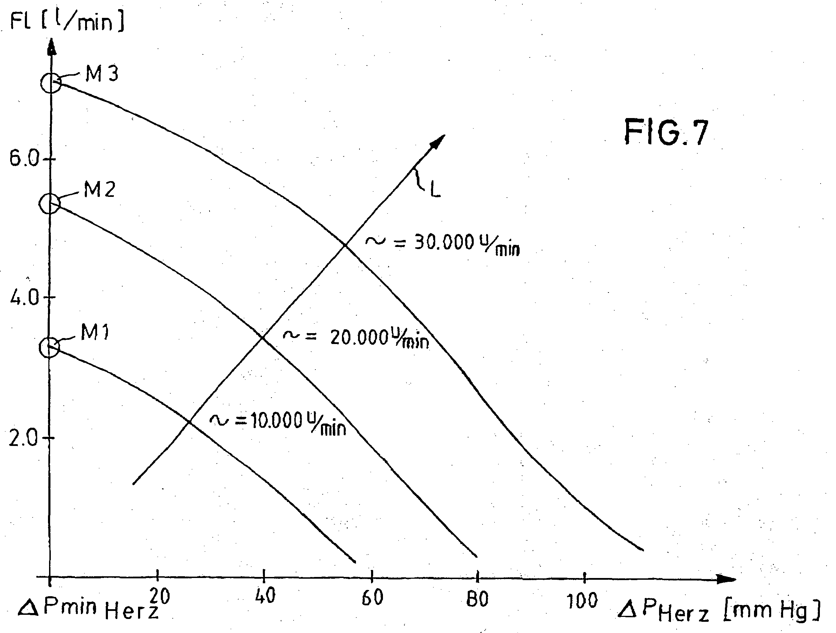

Maxima sind in Fig. 7 mit M1,M2,M3 bezeichnet. Bei geöffneter Aortenklappe AK ergibt sich somit bei eine Drehzahl von 10000 U/min ein Fluss von M1, bei einer Drehzahl von 20000 U/min ein Fluss von M2 und bei einer Drehzahl von 30000 U/min ein Fluss von M3. Diese Flussmaxima M1,M2,M3 für die verschiedenen Drehzahlen werden in einer Tabelle oder Kurve gespeichert und stellen die Soll-Flussmaxima dar. Zur Nachkalibrierung des Flusssensors wird bei einer beliebigen Drehzahl der Pumpe das Ist-Maximum des Flusses ermittelt und aus der Differenz zu dem Soll-Maximum wird ein Korrekturwert zur Driftkompensation gewonnen.

Auch bei der Rechtsherzpumpe, wie sie in Fig. 6 dargestellt ist, kann anstelle des Drucksensors 18 ein Flusssensor 20 benutzt werden. Hier wird bei stillstehender Pumpe der Fluss gemessen. Ergibt die Anzeige einen von Null abweichenden Fluss, so wird dieser als Korrekturwert für die Driftkorrektur benutzt.

## Patentansprüche

1. Intrakardiale Rotationspumpe (10) mit einem Drucksensor (18) und mit einem externen die Drehzahl eines die Pumpe (10) antreibenden Motors steuernden Steuergerät,
**gekennzeichnet durch**
- eine Tabelle oder Kurve von Soll-Druckminima (ΔP_{S}) in Abhängigkeit von der Drehzahl (n) der Pumpe (10) für einen intakten Drucksensor,
- Mittel zum Bestimmen eines Ist-Druckminimums (ΔP_{I}) des von dem Drucksensor (18) gemessenen Drucks bei einer betreffenden Drehzahl der Pumpe (10),
- Mittel zum Ermitteln der Drift des Drucksensors (18) aus der Differenz zwischen dem Ist-Druckminimum (ΔP_{I}) und dem der betreffenden Drehzahl entsprechenden Soll-Druckminimum (ΔP_{S}),
- Mittel zum Korrigieren jeweils nachfolgend gemessener Druckwerte durch Bestimmen eines Driftkorrekturwertes für die nachfolgend gemessenen Druckwerte aus der ermittelten Differenz zur Korrektur der Drift des Drucksensors (18) bei laufender Rotationspumpe (10).

2. Rotationspumpe nach Anspruch 1, **dadurch gekennzeichnet, dass** der Drucksensor (18) ein Differenzdrucksensor ist, der den Differenzdruck zwischen Außenseite und Innenseite eines rohrförmigen Pumpengehäuses (13) misst.

## Claims

1. An intracardiac rotary pump (10) having a pressure sensor (18) and an external control device controlling the rotational speed of a motor driving the pump (10),
**characterized by**
- a table or a curve of desired pressure minimum values (ΔP_{S}) as a function of the rotational speed (n) of the pump (10) for a functional pressure sensor,
- means for determining an actual pressure minimum value (ΔP_{I}) of the pressure measured by the pressure sensor (18) at a respective rotational speed of the pump (10),
- means for determining the drift of the pressure sensor (18) from the difference between the actual pressure minimum value (ΔP_{I}) and the desired pressure minimum value (ΔP_{S}) corresponding to the respective speed,
- means for correcting respective subsequently measured pressure values by determining a drift correction value for the subsequently measured pressure values on the basis of the determined difference from the correction of the drift of the pressure sensor (18) while the rotary pump (10) is running.

2. A rotary pump according to claim 1, **characterized in that** the pressure sensor (18) is a differential pressure sensor which measures the differential pressure between the outside and the inside of a tubular pump casing (13).

## Revendications

1. Pompe rotative intracardiaque (10) équipée d'un capteur de pression (18) et d'un dispositif de commande externe qui commande la vitesse d'un moteur d'entraînement de la pompe (10),
**caractérisée par**
- un tableau ou une courbe de minima de pression théorique (ΔPs) en fonction de la vitesse (n) de la pompe (10) pour un capteur de pression intact,
- des moyens pour déterminer un minimum de pression réelle (ΔP_{I}) de la pression mesurée par le capteur de pression (18) à une vitesse donnée de la pompe (10),
- des moyens pour déterminer la dérive du capteur de pression (18) à partir de la différence entre la pression minimale réelle (ΔP_{I}) et la pression minimale théorique (ΔPs) correspondant à la vitesse concernée,
- des moyens pour corriger des valeurs de pression respectivement mesurées par la suite par détermination d'une valeur de correction de dérive des valeurs de pression mesurées par la suite à partir de la différence déterminée pour la correction de la dérive du capteur de pression (18) lorsque la pompe rotative (10) est en marche.

2. Pompe rotative selon la revendication 1, **caractérisée en ce que** le capteur de pression (18) est un capteur de pression différentielle qui mesure la pression différentielle entre le côté extérieur et le côté intérieur d'un carter de pompe tubulaire (13).
